(19) Europäisches Patentamt
European Patent Office
Office européen des brevets

(11) Publication number: **0 226 406**
**B1**

(12) # EUROPEAN PATENT SPECIFICATION

(45) Date of publication of patent specification: **01.08.90**

(51) Int. Cl.$^5$: **C 07 D 241/52,** A 61 K 31/495

(21) Application number: **86309473.6**

(22) Date of filing: **04.12.86**

(54) **Process for purifying raw N-(2-hydroxyethyl)-2-methyl-3-carboxylic acid amido-quinoxalin-1,4-di-N-oxide.**

(30) Priority: **04.12.85 JP 273155/85**

(43) Date of publication of application:
**24.06.87 Bulletin 87/26**

(45) Publication of the grant of the patent:
**01.08.90 Bulletin 90/31**

(84) Designated Contracting States:
**CH DE ES IT LI**

(56) References cited:
**DE-A-2 035 480**
**FR-A-1 594 628**

(73) Proprietor: **Chisso Corporation**
**6-32, Nakanoshima 3-chome Kita-ku**
**Osaka-shi Osaka-fu (JP)**

(72) Inventor: **Harada, Hiroshi**
**598, Furuichiba**
**Ichiharashi Chibaken (JP)**
Inventor: **Fujita, Katsuhiko**
**3-444, Kofudai**
**Ichiharashi Chibaken (JP)**
Inventor: **Kawamura, Shinichi**
**5383-9 Goi**
**Ichiharashi Chibaken (JP)**

(74) Representative: **Lamb, John Baxter et al**
**MARKS & CLERK 57/60 Lincoln's Inn Fields**
**London WC2A 3LS (GB)**

**EP 0 226 406 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a process for purifying crude N-(2-hydroxyethyl)-2-methyl-3-carboxylic acid amido-quinoxalin-1,4-di-N-oxides; which compounds are useful as additives to feeding stuffs or medicines for animals.

DE—OS—2035400 discloses quinoxaline carboxamide-1,4-di-N-oxides of the formula

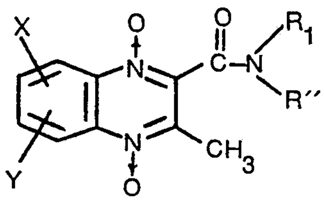

in which X is a hydrogen, methyl, methoxy, chlorine, fluorine, trifluoromethyl, sulphonamido, N-methylsulphonamide or N,N-dimethylsulphonamido substituent present in the 6 or 7 position, Y is a hydrogen, methyl, methoxy or chlorine substituent present in the 6 or 7 position.

$R_1$ is a hydrogen atom, methyl radical or ethyl radical, R'' is a lower alkyl—$NR_3R_4$ radical, a lower alkyl—$OR_5$ radical or a lower alkyl—$C(O)R_3R_6$ radical, in which $R_3$ is hydrogen, methyl or ethyl, $R_4$ is equal to $R_3$, provided that when $R_4$ is a lower alkanoyl radical $R_3$ should be hydrogen, $R_3$ and $R_4$, together with the nitrogen atom to which they are linked, can form a pyrrolo, pyrrolidino, piperazino, piperidino, lower N-alkylpiperazino or morpholino ring, $R_5$ is a hydrogen atom, lower alkyl radical or lower alkanoyl radical, $R_6$ is an hydroxy radical, lower alkoxy radical or —$NR_7R_8$, each $R_7$ and $R_8$ substituent being hydrogen, methyl or ethyl, $R_1$ and R'', together with the nitrogen atom to which they are linked, can form a piperazine ring of the formula:

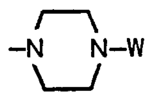

in which W is a hydrogen atom, a lower alkyl, lower alkanoyl, carbamoyl, lower carbamoylalkyl group.

On the other hand, the process disclosed in the specification of Japan Patent Application No. Sho 60—259249 resides in:

a process for producing a 2-methyl-3-carboxylic acid amidequinoxalin 1, 4-di-N-oxide of the formula

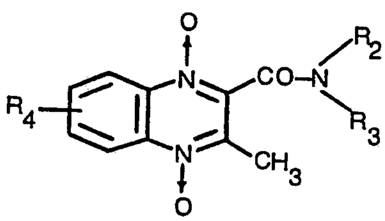

(3)

wherein $R_2$, $R_3$ and $R_4$ are defined below, which process comprises reacting an enamine of the formula

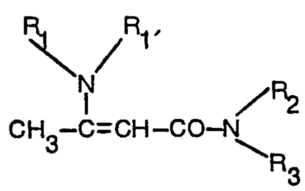

(1)

wherein $R_1$, $R_{1'}$, $R_2$ and $R_3$ each represent a linear or branched alkyl group which may be replaced by hydroxy, a lower alkoxy or alkoxycarbonyl group, or hydrogen, with a benzofluoxane of the formula

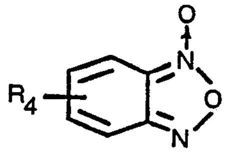

(2)

wherein $R_4$ represents hydrogen, a lower alkyl or lower alkoxy group, in an organic solvent in the presence of ammonia or a primary amine (Claim 1).

In the case of the compound of the above formula (A), either one of X or Y corresponds to hydrogen of the compound of the formula (3) and another thereof corresponds to $R_4$ of the formula (3), and $R_1$ and R'' correspond to $R_2$ and $R_3$ of the formula (3), respectively.

A process for purifying crude N-(2-hydroxyethyl)-2-methyl-3-carboxylic acid amido-quinoxalin-1,4-di-N-oxide has already been described in Japanese patent publication No. Sho 45—24988/1970. In this purification process, e.g. a mixed solvent comprising expensive dimethylformamide together with methanol is used as an organic solvent system for recrystallization. Hence it is desirable that the percentage recovery of the intended product (purified product) and the percentage recovery of the organic solvent through the recrystallization are both high.

However, as shown in the accompanying drawing, the solubility of N-(2-hydroxyethyl)-2-methyl-3-carboxylic acid amido-quinoxalin-1,4-di-N-oxide (hereinafter often simply referred to as "quinoxalin-1,4-di-N-oxide of the present invention") in a mixture of dimethylformamide methanol solvent is so low that the percentage recovery of the quinoxalin-1,4-di-N-oxide of the present invention obtained by recrystallization using the mixed solvent is also low. Whilst it may be contemplated to make the deposition temperature of the recrystallized crystals below 0°C, to thereby raise the percentage recovery, it is, however, disadvantageous, from the energy aspect, to cool a large quantity of solvent down to a temperature below 0°C, each time it is used.

Further, as to crude N-(2-hydroxyethyl)-2-methyl-3-carboxylic acid amido-quinoxalin-1,4-di-N-oxide prepared according to the known process described in Japanese patent publication No. Sho 45—24988, it has been found that even when it is recrystallized by the above mixed solvent, impurities which are difficult to remove still exist.

(Note: In order to basically solve the problem, the novel process for producing the quinoxalin-1,4-di-N-oxide as disclosed in our Japanese patent application No. Sho 60—259249 should be employed).

As a result of research in order to solve the above-mentioned problems of the prior art for purifying raw N-(2-hydroxyethyl)-2-methyl-3-carboxylic acid amido-quinoxalin-1,4-di-N-oxide, we have now found, in accordance with the present invention, that when recrystallizing purification is carried out using water as recrystallization solvent and under definite conditions, it is possible to obtain the quinoxalin-1,4-di-N-oxide of the present invention having a very low content of impurities in a high yield.

According to the invention, therefore, there is provided a process for purifying a N-(2-hydroxyethyl)-2-methyl-3-carboxylic acid amido-quinoxalin-1,4-di-N-oxide of the formula:

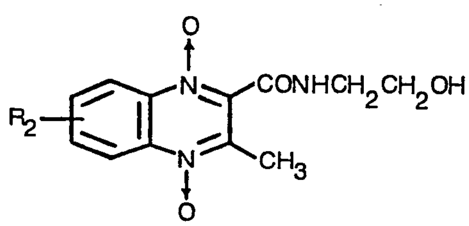

(3)

(wherein $R_2$ is a hydrogen atom or a lower alkyl or lower alkoxy group) which comprises dissolving the crude material in water at a temperature of 40° to 100°C and subsequently gradually cooling the resulting solution down to 0° to 30° to thereby effect recrystallization.

In the following description reference will be made to the accompanying drawings which is a graph illustrating the solubility (g/100 ml)-temperature (°C) curves of N-(2-hydroxyethyl)-2-methyl-3-carboxylic acid amido-quinoxalin-1,4-di-N-oxide in water and in known mixed dimethylformamide (DMF)/methanol (MCOH) solvents.

The crude N-(2-hydroxyethyl)-2-methyl-3-carboxylic acid amido-quinoxalin-1,4-di-N-oxide of formula (3) to be purified in accordance with the invention is obtained, by reacting a benzofuroxane of the formula:

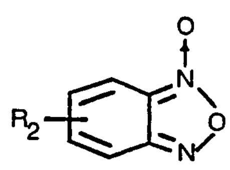

(1)

(in which $R_2$ has the meaning defined above) with an enamine of the formula:

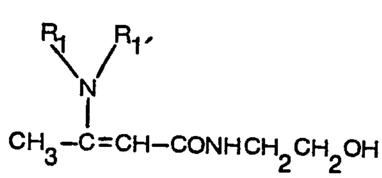

(2)'

in which $R_1$ and $R_{1'}$ are each a straight or branched alkyl group optionally substituted with a hydroxyl

group, a lower alkoxy group or an alkoxycarboxyl group, or H atom, in the presence of ammonia or a primary amine (a novel process disclosed in a patent application separately filed by the patent applicants).

For the recrystallization purification according to the invention, it is preferred to use a product obtained by the latter process, that is by the condensation of a benzofuroxane with an enamine, because the crude product prepared using an enamine is easier in the aspect of sufficient removal of impurities through recrystallization that is that prepared using an acetoacetic acid amide.

In the purification process of the invention, water is used as recrystallization solvent. Examples of polar organic solvents usable in admixture with water are lower alcohols such as methanol (meOH), ethanol, etc., dimethylformamide (DMF), dimethylsulphoxide, dioxane, etc.

The mixing proportion of water with these solvents has no particular limitation, but since the performance of water capable of dissolving the quinoxalin-1,4-di-N-oxide of the present invention is specifically high (see the drawing), it is preferred, even when using a mixture, to use water in a quantity of 50% by weight or more, preferably 70% by weight or more.

As is apparent from the drawing, the solubility of N-(2-hydroxyethyl)-2-methyl-3-carboxylic acid amido-quinoxalin-1,4-di-N-oxide (g/100 ml) is as follows: water at 0°C (0.13), mixed DMF/MeOH solvent (2:5) at 0°C (0.08), mixed DMF/MeOH solvent (1:5) at 0°C (0.05), water at 50°C (1.4), DMF/MeOH (2:5) at 50°C (0.55) and DMF/MeOH at 50°C (0.48). Thus, at the respective temperatures, the solubilities in water are several times greater than those in DMF/MeOH.

This fact indicates that as to the quantity of solvents required to recrystallize the same quantity of the quinoxalin-1,4-di-N-oxide of the present invention, the quantity in the case of water need only be a fraction of that in the case of DMF/MeOh. Further, the fact also means that as to water, it is possible to give weight rather in the recovery of the quinoxalin-1,4-di-N-oxide of the present invention from the part thereof dissolved in the solvent, than to recovery of the solvent.

The purification process of the present invention may be carried out in a conventional manner except that the substance to be purified and the solvent are specified. The concentration of the substance to be purified (the quinoxalin-1,4-di-N-oxide of the present invention) in solution has no particular limitation, but in the case of concentrations of 1 to 12 g/100 ml, purification is easily carried out. Further, the dissolution temperature also has no particular limitation, but in the case of 40° to 100°C, preferably 60° to 95°C, purification is easily carried out.

The temperature-reduction rate from the dissolution temperature down to the crystallization temperature is not restricted, but it is suitably from 160° to 2°C/hour, preferably from 80° to 5°C/hour. After crystallization, the crystallized product is separated from the mother liquor in a conventional manner, and if necessary, the crystallized product is further washed with a very small quantity of a low boiling solvent, followed by drying.

As described above, according to the process of the invention, it is not only possible to use very economical water as the solvent for purifying the quinoxalin-1,4-di-N-oxide of the present invention, but also since the solubility of the product to be purified in water is much higher than that in other known solvents, it is possible to raise, with a leap, the percentage recovery (yield) of the desired product based on the same quantity of the solvents.

The purification process of the present invention is particularly useful for the above compound for the above use applications. The present invention will be described in more detail by way of Examples.

### Example 1

An impurities-containing N-(2-hydroxyethyl)-2-methyl-3-carboxylic acid amido-quinoxalin-1,4-di-N-oxide (a raw reaction product after preparation) (80.0 g) was suspended in water (920 ml) and agitated, followed by heating to make the temperature of the resulting aqueous solution 90°C. When the aqueous solution was completely clarified, heating was stopped and the temperature was lowered from 90°C down to 0°C at a rate of 10°C/hour. Thereafter, pale yellow crystals were separated by suction-filtration and dried to obtain purified N-(2-hydroxyethyl)-2-methyl-3-carboxylic acid amido-quinoxalin-1,4-di-N-oxide.

Yield: 77.0 g (96.3%).

### Comparative Example 1

An impurities-containing N-(2-hydroxyethyl)-2-methyl-3-carboxylic acid amido-quinoxalin-1,4-di-N-oxide (e.g. a raw reaction product after its preparation) (13.0 g) was suspended in a mixed solvent of dimethylformamide-methanol (ratio by volume, 2:5) (987.0 ml) and agitated, followed by heating to make the temperature of the resulting solution 68°C (b.p.). When the solution was completely clarified, heating was stopped and the temperature was lowered from 68°C down to 0°C at a rate of 10°C/hour. Thereafter, pale yellow crystals were separated by suction-filtration and dried to obtained purified N-(2-hydroxyethyl)-2-methyl-3-carboxylic acid amido-quinoxalin-1,4-di-N-oxide.

Yield: 12.2 g (93.8%).

TABLE 1

Comparison of N-(2-hydroxyethyl)-2-methyl-3-carboxylic acid amido-quinoxalin-1,4-di-N-oxide obtained according to a known purification process with that obtained by purification according to the purification process of the present invention.

| | Purity (%) | Impurity (%) |
|---|---|---|
| Raw crystal | 99.51 | 0.33 |
| Purified product according to prior process | 99.77 | 0.08 |
| Purified product according to the present invention | 99.76 | 0.09 |

## Claims

1. A process for purifying a crude N-(2-hydroxyethyl)-2-methyl-3-carboxylic acid amido-quinoxalin-1,4-di-N-oxide of the formula:

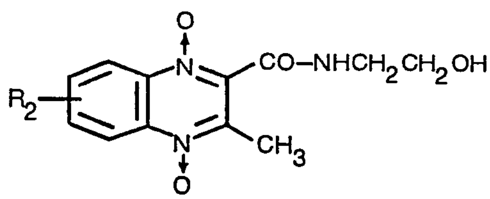

(3)

(in which $R_2$ is hydrogen atom or a lower alkyl or lower alkoxy group) obtained by reacting an enamine of the formula

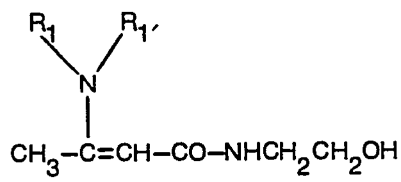

(I)

(in which $R_1$ and $R_{1'}$ each represent a linear or branched alkyl group optionally substituted with a hydroxyl group; or a lower alkoxy group or alkoxycarbonyl group) with a benzofuroxane of the formula:

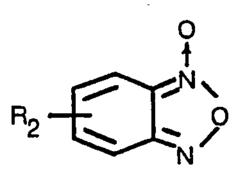

(2)

(in which $R_2$ has the meaning defined above) in the presence of ammonia or a primary amine; which purifying process comprises dissolving the crude material in water at a temperature of 40° to 100°C and subsequently gradually cooling the resulting solution down to 0° to 30°C, to thereby effect recrystallization.

2. A process as claimed in claim 1 in which the starting N-(2-hydroxyethyl)-2-methyl-3-carboxylic acid amido-quinoxalin-1,4-di-N-oxide is made into a hot aqueous solution having a concentration of 1 to 12 g/100 ml and subsequently cooled down to 0°C to 30°C.

3. A process as claimed in claim 2 in which the starting N-(2-hydroxyethyl)-2-methyl-3-carboxylic acid amido-quinoxalin-1,4-di-N-oxide is made into a hot aqueous solution thereof having a concentration of 2 to 9 g/100 ml at a temperature of 60° to 95°C, and subsequently cooled down to 0° to 15°C.

## Patentansprüche

1. Verfahren zur Reinigung von rohem 2-Methyl-3-[N-(2-hydroxyethyl)]- carbonsäureamido-chinoxalin-1,4-di-N-oxid der Formel:

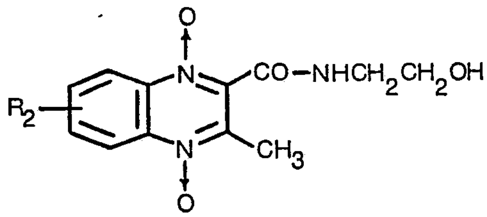

(3)

(worin $R_2$ ein Wasserstoffatom oder eine Niedrigalkyl- oder Niedrigalkoxygruppe ist), erhalten durch Umsetzen eines Enamins der Formel:

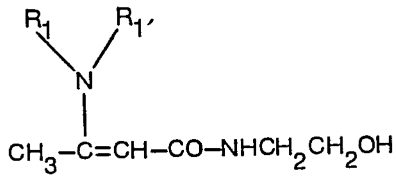

(1)

(worin $R_1$ und $R_{1'}$ jeweils eine geradkettige oder verzweigte, gegebenenfalls mit einer Hydroxylgruppe substituierte Alkylgruppe oder eine Niedrigalkoxygruppe oder Alkoxycarbonylgruppe bedeuten) mit einem Benzofuroxan der Formel:

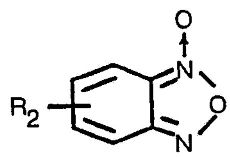

(2)

(worin $R_2$ die oben genannte Bedeutung besitzt) in Gegenwart von Ammoniak oder eines primären Amins, wobei das Reinigungsverfahren das Auflösen des Rohmaterials in Wasser bei einer Temperatur von 40) bis 100°C und nachfolgende schrittweise Abkühlen der entstandenen Lösung auf 0 bis 30°C hinab umfaßt, um dadurch das Umkristallisieren zu bewirken.

2. Verfahren nach Anspruch 1, bei dem das als Ausgangsmaterial verwendete 2-Methyl-3-[N-(2-hydroxyethyl)]- carbonsäureamido-chinoxalin-1,4-di-N-oxid in die Form einer heißen wäßrigen Lösung mit einer Konzentration von 1 bis 12 g/100 ml gebracht wird und anschließend auf 0 bis 30°C herabgekühlt wird.

3. Verfahren nach Anspruch 2, bei dem das als Ausgangsmaterial verwendete 2-Methyl-3-[N-(2-hydroxyethyl)]- carbonsäureamido-chinoxalin-1,4-di-N-oxid in die Form einer heißen wäßrigen Lösung mit einer Konzentration von 2 bis 9 g/100 ml bei einer Temperatur von 60 bis 95°C gebracht wird und anschließend auf 0 bis 15°C herabgekühlt wird.

**Revendications**

1. Procédé de purification de N-(2-hydroxyéthyl)-2-méthyl-quinoxaline-3-carboxamide-1,4-di-N-oxyde brut de formule:

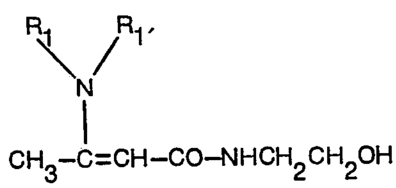

(1)

dans laquelle $R_2$ est un atome d'hydrogène ou un groupe alkyle inférieur ou alkoxy inférieur obtenu par réaction d'une énamine de formule:

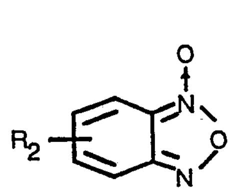

(2)

(dans laquelle $R_1$ et $R_{1'}$ représentent chacun un groupe alkyle linéaire ou ramifié éventuellement constitué

par un groupe hydroxyle: ou un groupe alkoxy inférieur ou un groupe alcoxycarbonyle) avec un benzofuroxane de formule:

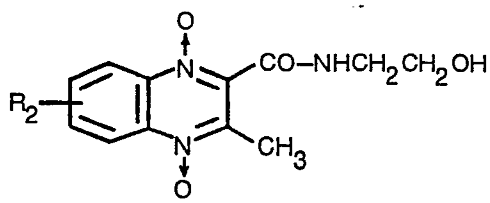

(3)

(dans laquelle $R_2$ a la signification ci-dessus) en présence d'ammoniac ou d'une amine primaire: procédé de purification comprenant la dissolution de la substance brute dans l'eau à une température de 40° à 100°C, puis le refroidissement progressif de la solution résultant jusqu'à 0° à 30°C, pour réaliser ainsi la recristallisation.

2. Procédé selon la revendication 1 dans lequel le N-(2-hydroxyéthyl)-2-méthyl-quinoxaline-3-carboxamide-1,4-di-N-oxyde de départ est amené sous forme d'une solution aqueuse chaude ayant une concentration de 1 à 12 g/100 ml puis refroidi jusqu'à 0°—30°C.

3. Procédé selon la revendication 2, dans lequel la N-(2-hydroxyéthyl)-2-méthyl-quinoxaline-3-carboxamide-1,4-di-N-oxyde de départ est amené sous forme d'une solution aqueuse chaude ayant une concentration de 2 à 9 g/100 ml à une température de 60 à 95°C, puis refroidi jusqu'à 0°—15°C.

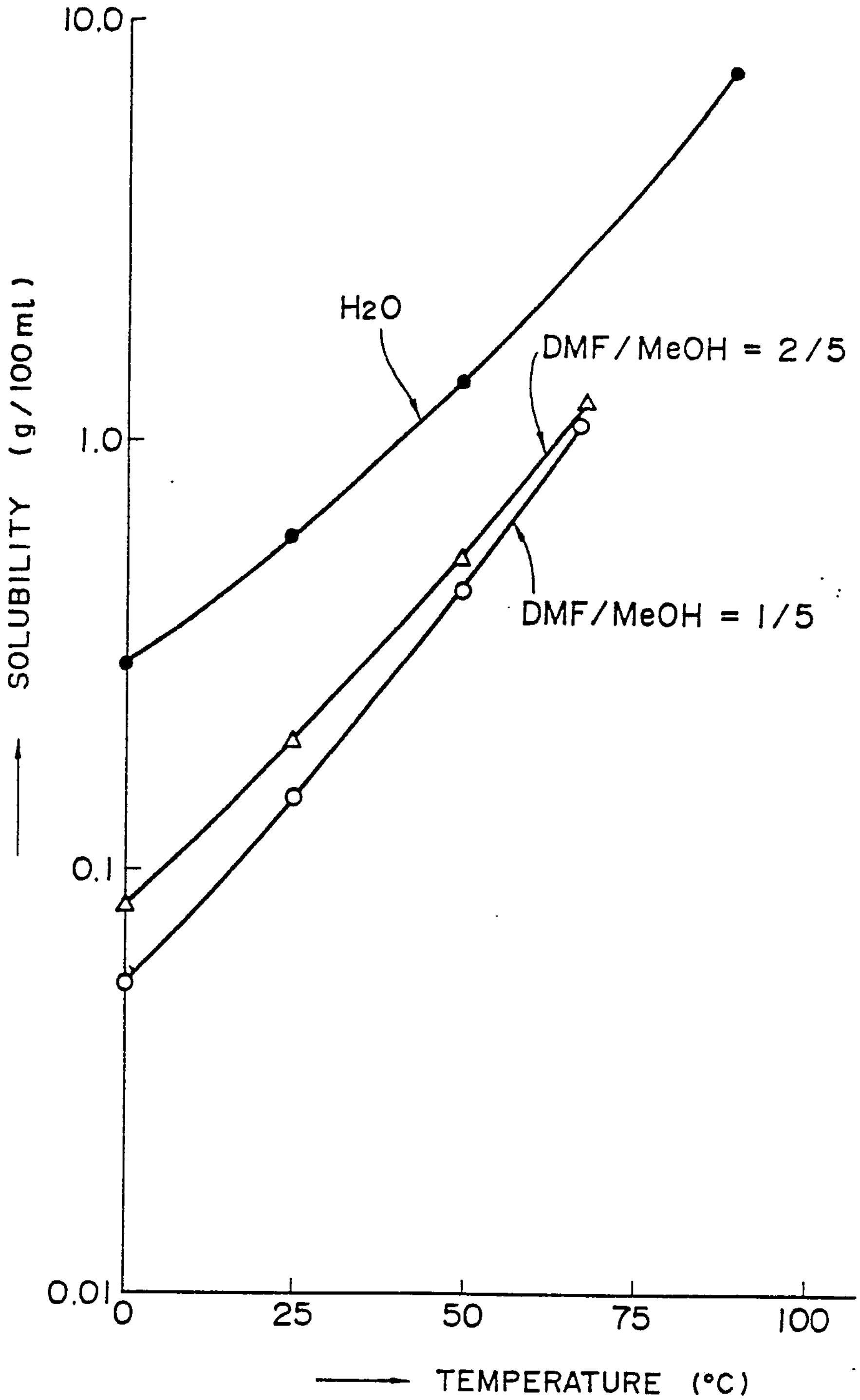
10.0
1.0
0.1
0.01
SOLUBILITY (g/100ml)
H2O
DMF/MeOH = 2/5
DMF/MeOH = 1/5
0
25
50
75
100
TEMPERATURE (°C)